Europäisches Patentamt

European Patent Office

Office européen des brevets

(11) Veröffentlichungsnummer: **0 310 685**
**A1**

## EUROPÄISCHE PATENTANMELDUNG

(21) Anmeldenummer: 87113166.0

(22) Anmeldetag: 09.09.87

(51) Int. Cl.4: **A61B 17/22 , A61B 17/32**

(43) Veröffentlichungstag der Anmeldung:
**12.04.89 Patentblatt 89/15**

(84) Benannte Vertragsstaaten:
**DE FR GB IT**

(71) Anmelder: **KONTRON-HOLDING AG**
**Bernerstrasse Süd 169**
**CH-8010 Zürich(CH)**

(72) Erfinder: **Marangoni, Daniele**
**26 Via Medici del Vascello**
**Mailand(IT)**
Erfinder: **Vassanelli, Corrado**
**Hospital Borgo Trento Cardiologia**
**Verona(IT)**

(74) Vertreter: **Buntz, Gerhard et al**
**Grenzacherstrasse 124 Postfach 3255**
**CH-4002 Basel(CH)**

(54) **Angioplastie-Katheter.**

(57) Vorrichtung zum Oeffnen von verengten oder verschlossenen Blutgefässen mit einem schlauchför-migen flexiblen Katheter (1), einem an dessen dista-lem Ende (2) angebrachten Bohrkopf (5) mit einem drehbaren Bohrwerkzeug (4) und mit Mitteln (7) zum zeitweiligen Verschliessen des Gefässes. Im Inneren des Katheters ist eine Welle (8) zum Antrieb des Bohrwerkzeugs und an seinem proximalen Ende (3) eine Antriebs-und Anschlusseinheit für den Antrieb der Welle und das Absaugen von abgetragenem Material angeordnet.

Fig.1

EP 0 310 685 A1

## Angioplastie-Katheter

Die Erfindung betrifft eine Vorrichtung zum Oeffnen von verengten oder verschlossenen Blutgefässen mit einem schlauchförmigen flexiblen Katheter.

Verfahren zur Aufdehung von Gefässen mit Hilfe von Kathetern werden üblicherweise als Angioplastie bezeichnet. Entsprechende Katheter werden demnach unter der Bezeichnung Angioplastie-Katheter zusammengefasst.

Während früher bei krankheitsverursachten Unterbrechungen des Blutkreislaufs die operative Umgehung der Unterbrechungsstelle häufig die einzige Möglichkeit war, wird in neuerer Zeit in zunehmendem Masse und mit zunehmendem Erfolg die Oeffnung der Gefässe durch Angioplastie erreicht. Die ersten Angioplastie-Verfahren beruhten auf der einfachen Aufdehnung einer Engstelle mit Hilfe eines Ballonkatheters, der die Engstelle mit verhältnissmässig hohem Druck aufdehnte. Dieses Verfahren hat den Nachteil, dass die Blutgefässe dem Druck teilweise elastisch nachgeben und sich später in verhältnissmässig kurzer Zeit wieder ihrer alten Form annähern. Die Oeffnung der Verengung ist daher nicht von Dauer.

Bei anderen Verfahren wird deshalb ein Teil der die Verengung hervorrufenden Ablagerung entfernt. Dies ist ohnehin immer dann nötig, wenn bereits ein totaler Verschluss vorliegt. Zu diesem Zweck wurden sowohl mecha nische, als auch mit Laserstrahlen arbeitende Verfahren vorgeschlagen.

In der DOS 32 42 341 ist ein Katheter beschrieben, an dessen distalem Ende sich ein Spreizkopf befindet, der ein oder mehrere Schälmesser aus elastischem Material aufweist, die mit Hilfe eines Spanndrahtes von einem am proximalen Ende angebrachten Griff aus ausgewölbt werden können. Mit dieser Ausstattung kann durch schabende Bewegungen der Einschluss, bzw. die Ablagerung, zerstört werden. Die abgetragenen Partikel werden durch eine Fördereinrichtung im Inneren des Katheters abgeführt. Diese Vorrichtung hat den Nachteil, dass die Schälmesser auch mit den Gefässwänden in Berührung kommen und diese verletzen können. Ausserdem besteht die Gefahr, dass abgetragene Partikel in der Blutbahn weggeschwemmt werden, wenn die Fördervorrichtung im Inneren des Katheters ihren Abtransport nicht bewerkstelligt, sodass es zu Embolien kommen kann.

Bei den mit Laserstrahlen arbeitenden Verfahren wird im Prinzip ein Kanal durch den verengenden Einschluss gebrannt. Der Nachteil dieses Verfahrens besteht darin, dass die innere Oberfläche des eingebrannten Kanals verhältnissmässig rauh bleibt und daher zur schnellen erneuten Verlegung neigt.

Der Erfindung liegt die Aufgabe zugrunde, einen neuen Angioplastie-Katheter vorzuschlagen, mit dem die Nachteile der bekannten Vorrichtungen dieser Art vermieden werden können.

Erfindungsgemäss wird dies erreicht durch einen Katheter der eingangs genannten Art, der sich durch einen an seinem distalen Ende angebrachten Bohrkopf mit einem drehbaren Bohrwerkzeug und mit Mitteln zum zeitweiligen Verschliessen des Gefässes, eine in seinem Inneren angeordnete Welle zum Antrieb des Bohrwerkzeugs und eine an seinem proximalen Ende angebrachte Antriebs- und Anschlusseinheit mit Antriebsmitteln zum Antrieb der Welle und Mitteln zum Absaugen von abgetragenem Material auszeichnet.

Nach einer besonderen Ausführungsform der Erfindung bestehen die Mittel zum zeitweiligen Verschliessen des Gefässes aus schirmartig aufspannbaren Flügeln, die im aufgespannten Zustand in Abhängigkeit von den Druckverhältnissen das Gefäss in der Art eines Rückschlagventils verschliessen oder öffnen.

Nach einer weiteren bevorzugten Ausführungsform der Erfindung besteht die Welle zum Antrieb des Bohrwerkzeugs aus einer im Inneren des Katheters angeordneten Spirale.

Eine weitere bevorzugte Ausführungsform der Erfindung weist als Antriebsmittel eine pneumatische Turbine auf, die zweckmässigerweise gleichzeitig zur Erzeugung von Unterdruck dient, mit dem das abgetragene Material abgesaugt wird.

Als Bohrwerkzeug kann nach der Erfindung bevorzugt eine Bohrkrone, eine Bürste, ein Schwamm, ein Schleifkopf oder ein Messer vorgesehen werden.

Gemäss einer weiteren bevorzugten Ausführungsform sind Mittel zum Einführen eines zusätzlichen Ballonkatheters vorgesehen, mit dessen Hilfe der Arbeitsbereich jenseits der Verengung verschlossen werden kann, um das Wegschwemmen von abgetragenen Partikeln vollständig auszuschliessen.

Im Folgenden werden bevorzugte Ausführungsbeispiele der Erfindung anhand der beiliegenden Zeichnungen beschrieben. Es zeigen:

Fig. 1 eine schematische Darstellung eines erfindungsgemässen Katheters im Schnitt im Zustand der Einführung.

Fig. 2 eine Darstellung des Katheters im arbeitsbereiten Zustand.

Fig. 3 eine schematische Darstellung des Bohrkopfes bei der Arbeit.

Fig. 4 alternativ zu Fig. 3 eine Version mit zusätzlich eingeführtem Ballonkatheter.

Fig. 5 eine Frontansicht des Bohrkopfes mit geschlossener Verschlussvorrichtung, d.h. im einführbereiten Zustand.

Fig. 6 eine Frontansicht des Bohrkopfes mit geöffneter Verschlussvorrichtung, d.h. im arbeitsbereiten Zustand.

Fig. 7-10 alternative Versionen des Bohrwerkzeugs.

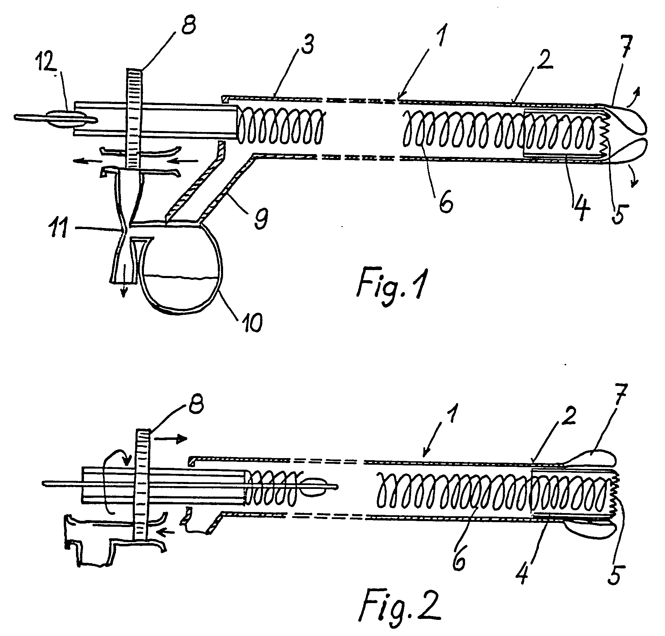

Fig. 1 zeigt einen Katheter 1, dessen langgestreckter Mittelteil gestrichelt angedeutet ist und der ein distales Ende 2 und ein proximales Ende 3 aufweist. Am distalen Ende 2 ist ein Bohrkopf 3 angebracht, der ein drehbar gelagertes Bohrwerkzeug 4 enthält. Das Bohrwerkzeug 4 besteht im wesentlichen aus einem hohlzylindrischen 3u Teil, an dessen einer Stirnseite eine scharfkantige Zähnung 5 vorgesehen ist. Fest mit dem Bohrwerkzeug 4 verbunden ist eine Spirale 6, die sich drehbar durch die Länge des ganzen Katheters 1 erstreckt und die zum Antrieb des Bohrwerkzeugs 4 dient.

Der Bohrkopf 3 weist ausserdem Mittel 7 auf, die in dem in Fig. 1 gezeigte einführbereiten Zustand die Stirnseite des Bohrwerkzeugs 4 abdekken. Diese Mittel 7 bestehen aus flügel- oder klappenähnlichen Teilen, die durch relative Verschiebung des Bohrkopfes gegenüber dem Katheter in den später gezeigten aufgespannten Zustand gebracht werden können.

Auf der proximalen Seite 3 des Katheters ist die Spirale 6 mit dem Antriebsrad 8 einer pneumatischen Turbine fest verbunden. Derartige Turbinen sind dem Fachmann an sich bekannt und müssen deshalb hier nicht im Detail beschrieben werden.

Das proximale Ende 3 des Katheters 1 weist ausserdem eine Verbindung 9 zu einem Absaugbehälter 10 auf. Dieses Absaugbehälter 10 ist wiederum mit einer Vakuumpumpe 11 verbunden, die in der vorliegenden Ausführungsform aus einem Venturirohr besteht, das mit der Turbine 8 in Verbindung ist.

Durch die Ausführung des Antriebs für das Bohrwerkzeug 4 in Form einer Spirale 6 bleibt ein innerer Hohlraum offen, durch den ein zusätzlicher Ballonkatheter 12 eingeführt werden kann, dessen Funktion später beschrieben wird.

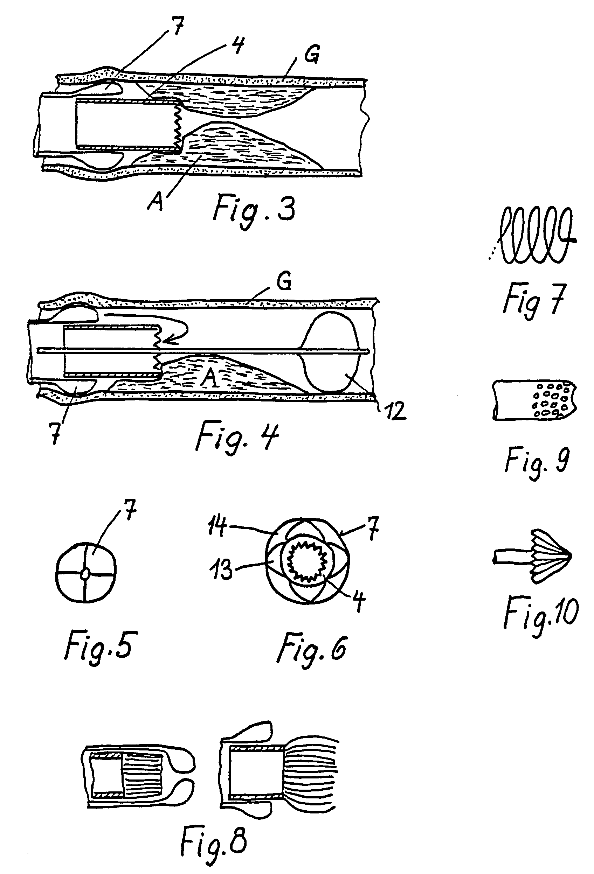

In Fig. 2 ist die Vorrichtung im arbeitsbereiten Zustand gezeigt. Das Bohrwerkzeug 4 ist relativ zum Katheter 1 nach vorne geschoben. Dadurch ist die Verschlussvorrichtung auseinander geklappt, sodass hinter dem Bohrkopf ein erweiterter Durchmesser entsteht. Das Aufklappen der Verschlussvorrichtung 7 ist aus den in Fig. 5 und 6 gezeigten Frontsichten erkennbar. Fig. 5 zeigt den eingeklappten Zustand, wie er in Fig. 1 zu sehen ist. Fig. 6 zeigt den aufgeklappten Zustand, wobei in dieser Darstellung der Aufbau der Verschlussvorrichtung erkennbar ist. Sie besteht aus vier flügel- oder blattähnlichen Teilen 13 die um 90° zueinander versetzt angeordnet sind und die untereinander durch flexible Zwischenwände 14 verbunden sind. Das Aufklappen bzw. Aufspannen erfolgt in der Art eines Schirms. Die Flügel 13 und die Zwischenwände 14 liegen mit ihrem äusseren Umfang an der Innenwand des Gefässes an, wenn die Verschlussvorrichtung aufgeklappt ist. Die Zwischenwände 14 sind flexibel. Wenn der Druck distal von der Verschlussvorrichtung höher ist als proximal, schliesst die Vorrichtung dicht ab. Umgekehrt klappen die Zwischenwände 14 bei niedrigerem distalem Druck ein und ermöglichen das Vorbeiströmen von Blut.

Gleichzeitig mit dem Vorschieben des Bohrwerkzeugs wird der Turbinenrotor 8 gegen den Gaseinlass der Turbine verschoben.

Fig. 3 zeigt den Bohrkopf 3 in der Arbeitsposition. Seine Aufgabe besteht darin, die auf der Innenseite einer Gefässwand G vorhandenen und das Gefäss einengenden Ablagerungen A zu beseitigen. Zu diesem Zweck wurde der Katheter in das Gefäss eingeführt bis der Bohrkopf 3 sich vor der Verengung befindet. Durch Vorschieben des Bohrwerkzeugs 4 wird die Verschlussvorrichtung 7 aufgespannt und das Gefäss auf diese Weise verschlossen. Dadurch kann beim nachfolgenden Bohren kein abgetragenes Material in der Zeichnung nach links weggeschwemmt werden. Das Bohrwerkzeug wird in Drehung mit hoher Drehzahl versetzt und weiter nach vorne geschoben. Dadurch wird ein Kanal durch die Ablagerung gebohrt bzw. gefräst. Die abgetragenen Partikel werden durch die gleichzeitige Saugwirkung durch den Katheter zu dessen proximalem Ende und in den Behälter 10 gesaugt.

Durch das dauernde Anliegen des Unterdrucks besteht an sich keine Gefahr, dass abgetragene Partikel in der Zeichnung nach rechts geschwemmt werden. Um aber das Gefäss auch in die andere Richtung abzudichten, kann der zusätzliche Ballonkatheter 12 durch den Bohrkopf nach aussen und auf die jenseitige Seite der Engstelle geschoben werden. Dort wird er aufgeblasen und schliesst das Gefäss nach der anderen Richtung ab.

In Fig. 4 ist ausserdem gezeigt, wie durch den Unterdruck, der infolge des Absaugens entsteht, ein gewisser Blutstrom an der Verschlussvorrichtung 7 vorbei in den Katheter gelangt.

Die Figuren 7-9 zeigen alternative Ausführungsformen des Bohr- bzw. Schneidwerkzeugs. Fig. 7 zeigt eine einfache Ausführungsform, bei der kein spezielles Bohrwerkzeug vorgesehen ist sondern das Ende der Spirale diese Funktion übernimmt. Dieses Spiralende ist zu diesem Zweck nach innen gebogen, um Verletzungen der Gefässwand zu vermeiden.

Fig. 8 zeigt ein bürstenförmiges Werkzeug, dass nach dem Herauschieben aus dem Katheter und infolge der Drehung einen erweiterten Durchmesser annimmt. Fig. 9 schliesslich zeigt ein Bohrwerkzeug mit einem schwammähnlichen Aufbau aus Karbonfasern. Das abgetragene Material wird durch die Oeffnungen ins Innere des Werkzeugs des Katheters gesaugt.

Fig. 10 zeigt ein schirm- oder pilzförmiges Werkzeug, das zunächst an der Verengung vorbei geschoben wird und das zu diesem Zweck vorzugsweise zusammenklappbar ist. Das Abtragen des abgelagerten Materials erfolgt beim Zurückziehen des Werkzeugs. Auf diese Weise wird das Wegschwemmen des abgetragenen Materials noch sicherer verhindert.

Der Vorteil der erfindungsgemässen Vorrichtung besteht darin, dass durch die sehr hohe Drehzahl, die mit der Turbine erreicht wird, eine äusserst glatte Oberfläche des gebohrten Kanals und eine hohe Arbeitsgeschwindigkeit erzielt wird. Auf diese Weise ist die Tendenz zu einem Neuverschluss und das Anginarisiko infolge des temporären Verschlusses während der Angioplastie stark verringert. Ausserdem ist durch das Verschliessen des Arbeitsbereichs in eine Richtung durch die Verschlussvorrichtung 7 und gegebenenfalls in andere Richtung durch den Ballonkatheter 12 die Gefahr des Wegschwemmens von abgelösten Partikeln eliminiert. Gleichzeitig wird auf diese Weise der Katheter im Gefäss zentriert, sodass auch die Gefahr einer Verletzung der Gefässwand infolge eines gegebenenfalls schräg geführten Werkzeugs stark verringert ist.

## Ansprüche

1. Vorrichtung zum Oeffnen von verengten oder ver. schlossenen Blutgefässen mit einem schlauchförmigen flexiblen Katheter, gekennzeichnet durch einen an seinem distalen Ende angebrachten Bohrkopf mit einem drehbaren Bohrwerkzeug und mit Mitteln zum zeitweiligen Verschliessen des Gefässes, eine in seinem Inneren angeordnete Welle zum Antrieb des Bohrwerkzeugs und eine an seinem proximalen Ende angebrachte Antriebs- und Anschlusseinheit mit Antriebsmitteln zum Antrieb der Welle und Mitteln zum Absaugen von abgetragenen Material auszeichnet.

2. Vorrichtung nach Anspruch 1, dadurch gekennzeichnet, dass die Mittel zum zeitweiligen Verschliessen des Gefässes aus schirmartig aufspannbaren Flügeln, die im aufgespannten Zustand in Abhängigkeit von den Druckverhältnissen das Gefäss in der Art eines Rückschlagventils verschliessen oder öffnen bestehen.

3. Vorrichtung nach einem der Ansprüche 1 und 2, dadurch gekennzeichnet, dass die Welle zum Antrieb des Bohrwerkzeugs aus einer im Inneren des Katheters angeordneten Spirale besteht.

4. Vorrichtung nach einem der Ansprüche 1-3, dadurch gekennzeichnet, dass die Antriebsmittel eine pneumatische Turbine aufweisen, die zweckmässigerweise gleichzeitig zur Erzeugung von Unterdruck dient, mit dem das abgetragene Material abgesaugt wird.

5. Vorrichtung nach einem der Ansprüche 1-4, dadurch gekennzeichnet, dass das Bohrwerkzeug eine Bohrkrone ist.

6. Vorrichtung gemäss einem der Ansprüche 1-4, dadurch gekennzeichnet, dass das Bohrwerkzeug eine Bürste ist.

7. Vorrichtung nach einem der Ansprüche 1-4, dadurch gekennzeichnet, dass das Bohrwerkzeug ein Schwamm ist.

8. Vorrichtung nach einem der Ansprüche 1-4, dadurch gekennzeichnet, dass das Bohrwerkzeug ein Schleifkopf ist.

9. Vorrichtung nach einem der Ansprüche 1-4, dadurch gekennzeichnet, dass das Bohrwerkzeug ein Messer ist.

10. Vorrichtung nach einem der Ansprüche 1-4, dadurch gekennzeichnet, dass das Bohrwerkzeug schirmförmig mit vorwärts gerichteter Spitze ausgebildet ist.

Fig.1

Fig.2

EP 0 310 685 A1

Fig. 3

Fig. 4

Fig. 5

Fig. 6

Fig 7

Fig. 9

Fig. 10

Fig. 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 086 048 (D. AUTH) <br> * Figuren 1,4; Seite 5, Zeile 24 – Seite 6, Zeilen 25; Anspruch 1 * | 1,3,8 | A 61 F 17/22 <br> A 61 F 17/32 |
| A | | 4 | |
| | --- | | |
| Y | FR-A-2 577 410 (KARCHER et al.) <br> * Zusammenfassung; Figur 1 * | 1,3,8 | |
| | --- | | |
| A | US-A-4 273 128 (B.G. LARY) <br> * Zusammenfassung; Figur 10 * | 9 | |
| | ----- | | |

**RECHERCHIERTE SACHGEBIETE (Int. Cl.4)**

A 61 F

Der vorliegende Recherchenbericht wurde für alle Patentansprüche erstellt

| Recherchenort | Abschlußdatum der Recherche | Prüfer |
|---|---|---|
| DEN HAAG | 09-05-1988 | NEILL M.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..................................................................................................
& : Mitglied der gleichen Patentfamilie, übereinstimmendes Dokument

EPO FORM 1503 03.82 (P0403)

**Europäisches Patentamt**

**EUROPÄISCHER RECHERCHENBERICHT**

Nummer der Anmeldung

EP 87 11 3166

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 086 048 (D. AUTH) <br> * Figuren 1,4; Seite 5, Zeile 24 - Seite 6, Zeilen 25; Anspruch 1 * | 1,3,8 | A 61 B 17/22 <br> A 61 B 17/32 |
| A | | 4 | |
| | --- | | |
| Y | FR-A-2 577 410 (KARCHER et al.) <br> * Zusammenfassung; Figur 1 * | 1,3,8 | |
| | --- | | |
| A | US-A-4 273 128 (B.G. LARY) <br> * Zusammenfassung; Figur 10 * | 9 | |
| | ----- | | |

**RECHERCHIERTE SACHGEBIETE (Int. Cl.4)**

A 61 B

Der vorliegende Recherchenbericht wurde für alle Patentansprüche erstellt

| Recherchenort | Abschlußdatum der Recherche | Prüfer |
|---|---|---|
| DEN HAAG | 09-05-1988 | NEILL M.C. |